# EUROPEAN PATENT SPECIFICATION

(11) **EP 2 633 322 B1**
(45) Date of publication and mention of the grant of the patent: **14.02.2018**
(21) Application number: 11773470.7
(22) Date of filing: 25.10.2011
(51) Int. Cl.: G01N 33/68

(54) **METHOD OF DETERMINING RISK OF ARRYTHMIA**
VERFAHREN ZUR BESTIMMUNG DES ARRYTHMIERISIKOS
PROCÉDÉ DE DÉTERMINATION DE RISQUE D'ARYTHMIE

(30) Priority: 19.11.2010 US 415368 P; 29.10.2010 US 407931 P
(43) Date of publication of application: 04.09.2013
(73) Proprietor: F. Hoffmann-La Roche AG, 4070 Basel (CH)
(72) Inventor: ABRAMS, Rory, Brooklyn, New York 11210 (US); BABIARZ, Joshua, Belleville, New Jersey 07109 (US); CHIAO, Eric, Chatham, New Jersey 07928 (US); GUO, Liang, New Hyde Park, New York 11040 (US); KOLAJA, Kyle, L., Montclair, New Jersey 07042 (US)
(74) Representative: Townsend, Carolin
(86) International application number: PCT/EP2011/068579
(87) International publication number: WO 2012/055828

(56) References cited:
- REPPEL MICHAEL ET AL: "Effect of cardioactive drugs on action potential generation and propagation in embryonic stem cell-derived cardiomyocytes", CELLULAR PHYSIOLOGY AND BIOCHEMISTRY, vol. 19, no. 5-6, 2007, pages 213-224, XP002666063, ISSN: 1015-8987
- CASPI OREN ET AL: "In vitro electrophysiological drug testing using human embryonic stem cell derived cardiomyocytes.", STEM CELLS AND DEVELOPMENT 2009 JAN-FEB LNKD- PUBMED:18510453, vol. 18, no. 1, January 2009 (2009-01), pages 161-172, XP002666064, ISSN: 1557-8534
- ASAI YASUYUKI ET AL: "Combination of functional cardiomyocytes derived from human stem cells and a highly-efficient microelectrode array system: an ideal hybrid model assay for drug development.", CURRENT STEM CELL RESEARCH & THERAPY SEP 2010 LNKD- PUBMED:20214558, vol. 5, no. 3, September 2010 (2010-09), pages 227-232, XP9155001, ISSN: 1574-888X
- LIANG HUAMIN ET AL: "Human and murine embryonic stem cell-derived cardiomyocytes serve together as a valuable model for drug safety screening.", CELLULAR PHYSIOLOGY AND BIOCHEMISTRY : INTERNATIONAL JOURNAL OF EXPERIMENTAL CELLULAR PHYSIOLOGY, BIOCHEMISTRY, AND PHARMACOLOGY 2010 LNKD- PUBMED:20332627, vol. 25, no. 4-5, 23 March 2010 (2010-03-23), pages 459-466, XP002666065, ISSN: 1421-9778
- YOKOO N ET AL: "The effects of cardioactive drugs on cardiomyocytes derived from human induced pluripotent stem cells", BIOCHEMICAL AND BIOPHYSICAL RESEARCH COMMUNICATIONS, ACADEMIC PRESS INC. ORLANDO, FL, US, vol. 387, no. 3, 25 September 2009 (2009-09-25), pages 482-488, XP026448304, ISSN: 0006-291X, DOI: 10.1016/J.BBRC.2009.07.052 [retrieved on 2009-07-16]
- FARKAS ATTILA S ET AL: "Minimizing repolarization-related proarrhythmic risk in drug development and clinical practice.", DRUGS 26 MAR 2010 LNKD- DOI:10.2165/11535230-000000000-00000 PUBMED:20329805, vol. 70, no. 5, 26 March 2010 (2010-03-26) , pages 573-603, XP9155000, ISSN: 0012-6667
- GUO LIANG ET AL: "Estimating the risk of drug-induced proarrhythmia using human induced pluripotent stem cell-derived cardiomyocytes.", TOXICOLOGICAL SCIENCES : AN OFFICIAL JOURNAL OF THE SOCIETY OF TOXICOLOGY SEP 2011 LNKD- PUBMED:21693436, vol. 123, no. 1, September 2011 (2011-09), pages 281-289, XP9154987, ISSN: 1096-0929
- GUO LIANG ET AL: "The electrophysiological effects of cardiac glycosides in human iPSC-derived cardiomyocytes and in guinea pig isolated hearts.", CELLULAR PHYSIOLOGY AND BIOCHEMISTRY : INTERNATIONAL JOURNAL OF EXPERIMENTAL CELLULAR PHYSIOLOGY, BIOCHEMISTRY, AND PHARMACOLOGY 2011 LNKD- PUBMED:21691062, vol. 27, no. 5, 15 June 2011 (2011-06-15), pages 453-462, XP002666067, ISSN: 1421-9778

## Description

The present invention relates to a method of determining the risk of drug induced arrhythmia using stem cell derived cardiomyocytes in a high-throughput impedance or multi-electrode array assay.

### BACKGROUND OF THE INVENTION

Drug-induced Torsades de Pointes (TdP), a life threatening polymorphic ventricular tachyarrhythmia, has led to the withdrawal or severe limitation of the use of a number of drugs *(*Journal of Pharmacological and Toxicological Methods 52: 46-59, 2005). The typical cause of TdP is inhibition of the inward rectifying potassium channel, hERG (*human ether-a-go-go* related gene encoded by *KCNH2*), resulting in a prolonged QT interval. Currently, standard approach is to screen drug candidates for hERG inhibition utilizing non-cardiac cell lines overexpressing hERG. However, hERG screening is suboptimal, as not all compounds that inhibit the hERG channel result in QT prolongation or induce TdP (Cardiovascular Research 58: 32-45, 2003). Furthermore, arrhythmias can be induced in humans by drugs that do not exhibit *in vitro* hERG inhibition or do not cause QT prolongation in pre-clinical animal models. For example, verapamil, a potent hERG inhibitor, does not cause QT prolongation or TdP in patients and is used safely and effectively. Despite this discordance, extensive time and effort in drug discovery is spent on investigating a compound's effect on hERG inhibition and QT interval prolongation as a surrogate for human TdP potential, largely due to a general lack of refined alternatives.

Technologies such as multi-electrode arrays (MEA) can be used to measure field potential and, when examining a homogenous and electrically coupled population of cells, mimic in vivo electrocardiograms (Journal of Electrocardiology 37 Suppl: 110-116, 2004). MEAs have been used previously to assess the general properties of stem cell derived cardiomyocyte action potential and the electrophysiological changes can be used as a surrogate measure of arrhythmias. However, technical limitations restrict the throughput and duration of MEA studies *(*Stem Cell Research 4: 107-116; Stem Cell Research 4: 189-200)). Beyond hERG inhibition, the most frequently studied models for interrogating TdP potential are low-throughput *ex vivo* models such as Langendorff preparations and ventricular wedge (Journal of Pharmacological and Toxicological Methods 52: 46-59, 2005). Reppel M et al, 2007 Cellular Physiology and Biochemistry 19(5-6):213-224 relates to the effect of cardioactive drugs on action potential generation and propagation in embryonic stem cell-derived cardiomyocytes. Caspri O et al, 2009 Stem Cells and Development 18(1):161-172 relates to *in vitro* electrophysiological drug testing using human embryonic stem cell derived cardiomyocytes. Asai Y et al, 2010 Current Stem Cell Research & Therapy 5(3):227-232 relates to combination of functional cardiomyocytes derived from human stem cells and a highly-efficient microelectrode array system. Liang H et al, 2010 Cellular Physiology and Biochemistry 25(4-5):459-466 relates to human and murine embryonic stem cell-derived cardiomyocytes and their value for drug safety screening. Yokoo N et al, 2009 Biochemical and Biophysical Research 387(3):482-488 relates to the effect of cardioactive drugs on cardiomyocytes derived from human induced pluripotent stem cells. Farkas A et al, 2010 Drugs 70(5):573-603 relates to minimizing repolarization-related proarrhythmic risk in drug development and clinical practice.

Improved *in vitro* systems for predicting drug-induced toxicities are needed by the pharmaceutical and biotechnology industries to decrease late-stage drug attrition. Specifically, there is need for a high-throughput *in vitro* model that goes beyond QT prolongation and delves directly into the functional interplay of the multiple ion channels used by the human cardiomyocyte to coordinate normal rhythmic contractions.

### SUMMARY OF THE INVENTION

Herein, we disclose the first high-throughput functional assay employing human induced pluripotent stem cell-derived cardiomyocytes (iPSC-CM) that accurately detects drug-induced cardiac abnormalities. Two independent methods were used, one of 96-well plates with interdigitated electrode sensor arrays measuring impedance and a second method using multi-electrode arrays. Impedance due to the adherent iPSC-CMs was sampled every 12.9 milliseconds, giving a non-destructive, continuous measurement of the physical attachment of the cells to the dish. Traces of the impedance revealed rhythmic contractions of the iPSC-CM. Treatment with a wide range of drugs known to alter cardiac function induced changes in beat rate and contractility detected by impedance. Similar results for drug effects were collected on micro-electrode arrays thus confirmed the validity of the model. Drug-induced arrhythmias were detected, quantified, and a prediction of proarrhythmic potential calculated (PPS; predicted proarrhythmia score), illustrating this system's ability to interrogate the functional properties of iPSC-CM in a manner previously not possible, providing a new opportunity for predictive cardiovascular safety.

The present application provides a method of determining risk of drug-induced arrhythmia comprising:
a) providing cardiomyocytes;
b) contacting said cardiomyocytes with said drug;
c) detecting beat rate irregularity by monitoring either cell contraction or field potential.

The present application provides the above method, wherein the cardiomyocytes are of human origin.

The present application provides the above methods, wherein the cardiomyocytes are produced from a pluripotent stem cell source.

The present application provides the above methods, wherein the stem cell source is an induced pluripotent stem cell source.

The present application provides the above methods, wherein the incidence of arrhythmia is detected by monitoring changes in cell field potential.

The present application provides the above methods, wherein the cell contraction is monitored by measuring impedance.

The present application provides the above methods, wherein the cell contraction is monitored by measuring impedance at a data capture rate frequency capable of identifying the movement of cardiomyocytes during contraction.

The present application provides the above methods, wherein the detection of arrhythmia comprises monitoring increases, decreases, or irregularity of beat rate rhythm.

The present application provides the above methods, wherein further comprising:
d) calculating theIBR.

The present application provides the above methods, wherein a IBR of less than or equal to 0.2 is used to designate a low risk of arrhythmia.

The present application provides the above methods, wherein method above, further comprising:
e) calculating the ratio of the IB₂₀ relative to the Cₘₐₓ, resulting in a PPS or calculating the ratio of the IB₂₀ relative to Cₘₐₓ, resulting in a PPS.

The present application provides the above methods, wherein a PPS of less than or equal to 100 is used to designate a low risk of arrhythmia.

The present application provides the above methods, wherein the method of determining risk of drug-induced arrhythmia is conducted in a high throughput format.

The present application provides the above methods, wherein the method of determining risk of drug-induced arrhythmia is conducted to screen molecules in a drug development setting.

### DETAILED DESCRIPTION OF THE INVENTION

The present application provides a method of determining risk of drug-induced arrhythmia comprising:
a) providing cardiomyocytes;
b) contacting said cardiomyocytes with said drug;
c) detecting beat rate irregularity by monitoring either cell contraction or field potential.

The present application provides the above method, wherein the cardiomyocytes are of dog, monkey, rat, rabbit, or human origin.

The present application provides the above methods, wherein the cardiomyocytes are of human origin.

The present application provides the above methods, wherein the cardiomyocytes are produced from a stem cell source.

The present application provides the above methods, wherein the stem cell source is an embryonic stem cell source.

The present application provides the above methods, wherein the cardiomyocytes are produced from a pluripotent stem cell source.

The present application provides the above methods, wherein the stem cell source is an induced pluripotent stem cell source.

The present application provides the above methods, wherein the incidence of arrhythmia is detected by monitoring changes in cell field potential.

The present application provides the above methods, wherein the cell contraction is monitored by measuring impedance.

The present application provides the above methods, wherein the cell contraction is monitored by measuring impedance at a data capture rate frequency capable of identifying the movement of cardiomyocytes during contraction.

The present application provides the above methods, wherein the detection of arrhythmia comprises monitoring increases, decreases, or irregularity of beat rate rhythm.

The present application provides the above methods, wherein the irregularity of beat rate rhythm is indicative of Torsades de Pointes.

The present application provides the above methods, wherein the irregularity of beat rate rhythm is indicative of prolongation of QT.

The present application provides the above methods, wherein the irregularity of beat rate rhythm is due to disruption of a cardiac ion channel.

The present application provides the above methods, wherein the impedance is measured with a sampling rate of about every 12.9 milliseconds.

The present application provides the above methods, wherein further comprising:
d) calculating IB₂₀.

The present application provides the above methods, wherein a IBR of less than or equal to 0.2 is used to designate a low risk of arrhythmia.

The present application provides the above methods, wherein method above, further comprising:
e) calculating the ratio of the IB₂₀ relative to the Cₘₐₓ, resulting in a PPS or calculating the ratio of the IB₂₀ relative to Cₘₐₓ, resulting in a PPS .

The present application provides the above methods, wherein further comprising:
e) calculating the ratio of the Cₘₐₓ relative to the IB₂₀, resulting in a PPS.

The present application provides the above methods, wherein a ratio of the IB₂₀ relative to the Cₘₐₓ of less than or equal to 100 is used to designate a low risk of arrhythmia.

The present application provides the above methods, further comprising:
f) comparing the result of step e) to that of a known arrhythmia inducing drug.

The present application provides the above methods, wherein the cardiac ion channel is a potassium channel.

The present application provides the above methods, wherein the potassium channel is the hERG channel.

The present application provides the above methods, wherein the cardiac ion channel is a calcium channel.

The present application provides the above methods, wherein the cardiac ion channel is a sodium channel.

The present application provides the above methods, wherein the method of determining risk of drug-induced arrhythmia is conducted in a high throughput format.

The present application provides the above methods, wherein the method of determining risk of drug-induced arrhythmia is conducted to screen molecules in a drug development setting.

It is understood that the methods provided herein are in vitro methods, i.e. are not performed on the human or animal body.

The capability to direct the differentiation of pluripotent stem cells (PSC) into beating, maturing cardiomyocytes affords a novel path to study cardiovascular biology *in vitro (*Trends in Pharmacological Sciences 30: 536-545, 2009). Further, human PSC-based predictive toxicity assays can help calibrate the potential safety issues of promising drug candidates early in the development process and provide insight into the mechanisms of drug-induced organ toxicity, all while reducing, refining, and replacing the reliance on live animal testing. Using karyotypically normal human PSC derived-tissues could increase the relevance and predictive value of pre-clinical safety assessment, since traditional approaches rely heavily on animal models that marginally predict human responses and offer limited ability to refute false positives *(*Regul. Toxicol. Pharmacol. 32: 56-67, 2000). Furthermore, a panel of PSCs with defined human allelic variations would help identify patient subpopulations that exhibit varied drug responses, aiding in the optimization of the inclusion and exclusion criteria key to successful clinical trial design.

The approach described herein uses a 96-well tissue culture plate with an interdigitated electrode sensor array capable of rapid sampling of impedance. Previously published uses of impedance measurements in cell culture relied on a slower sampling rate and generally were limited to measuring general cellular effects such as cytotoxicity or motility (Nature 366: 591-592, 1993, Biotechnology Journal 3: 484-495, 2008). By increasing the sampling rate to 12.9 milliseconds, the physical movement of contracting cardiomyocytes can be observed. This allows real-time, label-free monitoring of the rhythmic contraction of living, human iPSC-derived cardiomyocytes. The direct measure of the functional contraction of human cardiomyocytes makes possible the high-throughput *in vitro* screening of the pro-arrhythmic potential of novel molecular entities.

Because the use of rapid impedance sampling has not been applied to detecting spontaneous cardiomyocyte beating, we first sought to fully characterize the system. The beat rate of untreated iPSC-CMs consistently averaged ∼40 beats per minutes (BMP) across individual wells and plates, as measured by both MEA and impedance. To establish that the rapid impedance readings reflect the physical contraction of the cells as opposed to the electrical changes, the effects of the myosin II inhibitor, blebbistatin, were examined. Following treatment with blebbistatin no cardiomyocyte beating was observed using impedance, yet the electrophysiology of the cardiomyocytes detected by MEA remained unchanged, confirming that impedance is indeed directly measuring the physical movement of the cardiomyocyte during contraction and not measuring artifacts related to the electrical field of the voltage-dependent cardiac action potential.

Next we sought to systematically examine the effects of different classes of cardioactive drugs (Tables I-II).

IBR stands for "Irregular Beat Ratio", calculated as the number of irregular beats divided by the total beats over one minute (= irregular beats/total beats). IBR will be a value between 0-1 (or 0-100% if using % as the unit). IB₂₀ is equal to the lowest concentration that produces an IBR value ≥ 0.2 (≥ 20% irregular beats over one minute). In this application, PPS is calculated by two different ways:
1) Cₘₐₓ/IB₂₀, the ratio of Cₘₐₓ relative to IB₂₀ and the cut-off is >100 as a level of concern (Table I):

**Table I.**

| **Drug** | **Cₘₐₓ (nM)** | **IB₂₀ (µM)*** | **PPS** | **hERG** | **QT** | **TdP** |
|---|---|---|---|---|---|---|
| **Ouabain** | **170** | **0.03** | **5667** | **(-)** | **(-)** | **(+)** |
| **Aconitine** | **77** | **0.03** | **2567** | **(-)** | **(-)** | **(+)** |
| **Quinidine** | **21,578** | **10** | **2158** | **(+)** | **(+)** | **(+)** |
| **Dofetilide** | **6** | **0.003** | **2000** | **(+)** | **(+)** | **(+)** |
| **Flecainide** | **1,931** | **1** | **1931** | **(+)** | **(+)** | **(+)** |
| **Erythromycin** | **34,064** | **30** | **1135** | **(+)** | **(+)** | **(+)** |
| **Terfenadine** | **300** | **0.3** | **1000** | **(+)** | **(+)** | **(+)** |
| **Thioridazine** | **1781** | **3** | **594** | **(+)** | **(+)** | **(+)** |
| **RO5657** | **5,548** | **10** | **555** | **(+)** | **(+)** | **(+)** |
| **Sotalol** | **14,733** | **30** | **491** | **(+)** | **(+)** | **(+)** |
| **E-4031** | **13** | **0.03** | **433** | **(+)** | **(+)** | **(+)** |
| **Cisapride** | **129** | **0.3** | **429** | **(+)** | **(+)** | **(+)** |
| **Astemizole** | **8** | **0.03** | **262** | **(+)** | **(+)** | **(+)** |
| **Ranolazine** | **6,009** | **100** | **60** | **(+)** | | **(-)** |
| **Alfuzosin** | **56** | **1** | **56** | **(-)** | **(+)** | **(-)** |
| **Moxifloxacin** | **10,276** | **>100** | **<103** | **(+)** | **(+)** | **(±)** |
| **Nifedipine** | **194** | **>3** | **<65** | **(-)** | **(-)** | **(-)** |
| **Amiodarone** | **3874** | **>100** | **<39** | **(+)** | **(+)** | **(±)** |
| **Verapamil** | **815** | **>30** | **<27** | **(+)** | **(±)** | **(-)** |
| **Captopril** | **2,466** | **>100** | **<25** | **(-)** | **(-)** | **(-)** |
| **Amoxicillin** | **17,036** | **>1,000** | **<17** | **(-)** | **(-)** | **(-)** |
| **Fluoxetine** | **485** | **>30** | **<16** | **(+)** | **(+)** | **(-)** |
| **Rofecoxib** | **1021** | **>100** | **<10** | **(-)** | **(-)** | **(-)** |
| **Aspirin** | **10,000** | **>1,000** | **<10** | **(-)** | **(-)** | **(-)** |

2) IB₂₀/Cₘₐₓ, the ratio of IB₂₀ relative to Cₘₐₓ, with the cut-off of < 10 as a level of concern (Table II):

**Table II**

| **Drug** | **Cₘₐₓ (nM)** | **IB₂₀ (nM)*** | **PPS** | **hERG** | **QT** | **TdP** |
|---|---|---|---|---|---|---|
| **Ouabain** | **170** | **30** | **0.2** | **(-)** | **(-)** | **(+)** |
| **Aconitine** | **77** | **30** | **0.4** | **(-)** | **(-)** | **(+)** |
| **Quinidine** | **21,578** | **10,000** | **0.5** | **(+)** | **(+)** | **(+)** |
| **Dofetilide** | **6** | **3** | **0.5** | **(+)** | **(+)** | **(+)** |
| **Flecainide** | **1,931** | **1,000** | **0.5** | **(+)** | **(+)** | **(+)** |
| **Erythromycin** | **34,064** | **30,000** | **0.9** | **(+)** | **(+)** | **(+)** |
| **Terfenadine** | **300** | **300** | **1.0** | **(+)** | **(+)** | **(+)** |
| **Thioridazine** | **1781** | **3,000** | **1.7** | **(+)** | **(+)** | **(+)** |
| **RO5657** | **5,548** | **10,000** | **1.8** | **(+)** | **(+)** | **(+)** |
| **Sotalol** | **14,733** | **30,000** | **2.0** | **(+)** | **(+)** | **(+)** |
| **E-4031** | **13** | **30** | **2.3** | **(+)** | **(+)** | **(+)** |
| **Cisapride** | **129** | **300** | **2.3** | **(+)** | **(+)** | **(+)** |
| **Astemizole** | **8** | **30** | **3.8** | | **(+)** | **(+)** |
| **Ranolazine** | **6,009** | **100,000** | **17** | **(+)** | **(+)** | **(-)** |
| **Alfuzosin** | **56** | **1,000** | **18** | **(-)** | **(+)** | **(-)** |
| **Moxifloxacin** | **10,276** | **>100,000** | **>10** | **(+)** | **(+)** | **(±)** |
| **Nifedipine** | **194** | **>3,000** | **>16** | **(-)** | **(-)** | **(-)** |
| **Amiodarone** | **3874** | **>100,000** | **>26** | **(+)** | **(+)** | **(±)** |
| **Verapamil** | **815** | **>30,000** | **>37** | **(+)** | **(±)** | **(-)** |
| **Captopril** | **2,466** | **>100,000** | **>41** | **(-)** | **(-)** | **(-)** |
| **Amoxicillin** | **17,036** | **>1,000,000** | **>59** | **(-)** | **(-)** | **(-)** |
| **Fluoxetine** | **485** | **>30,000** | **>62** | **(+)** | **(+)** | **(-)** |
| **Rofecoxib** | **1021** | **>100,000** | **>98** | **(-)** | **(-)** | **(-)** |
| **Aspirin** | **10,000** | **>1,000,000** | **>100** | **(-)** | **(-)** | **(-)** |

The 2^{nd} way of calculation (IB₂₀/Cₘₐₓ) is commonly used in the field of safety assessment and termed as the "Safety Margin", which indicates how close the concentration that causes the safety concern to the concentration of therapeutic efficacy in the clinic. The larger the safety margin is, the lower the risk for drug toxicity. Conversely, using the 1^{st} ratio, (Cₘₐₓ/IB₂₀), the larger the PPS value is, the higher the risk is for drug toxicity.

Tetrodotoxin, a pure Na⁺ channel blocker isolated from puffer fish toxin and ZD7288, a blocker of pace-maker current (If), both exhibited a reduction in beat rate as predicted. Isoproterenol, an agonist of β-adrenergic receptor increased both beat rate and amplitude of impedance, and beat rate in MEA. Ouabain, a positive inotropic agent which blocks the K⁺/Na⁺-ATPase raising intracellular Na⁺ and Ca²⁺, increased amplitude of both MEA and impedance measurements. Treatment with nifedipine, a specific blocker of L-type Ca²⁺ channel, resulted in the expected decrease in the amplitude of each beat in impedance and Ca²⁺-peak amplitude in MEA coupled with an increase in beat rate. As negative controls, compounds such as aspirin, amoxicillin, and captopril, which are devoid of cardiovascular side effects *in vivo,* did not show any alterations in beat patterns within human iPSC-CMs. Taken together, these results demonstrate that rapid impedance measurement of iPSC-CMs can detect the appropriate and expected responses for receptor/transporter modulation and ion channel inhibition.

Following treatment with the hERG inhibitor, E4031, a dose-dependent reduction in beat rate as well as instances of erratic impedance changes were observed, resembling afterdepolarizations and arrhythmias (Figure 1.). As ventricular arrhythmic beats are considered to be initiated by altered Ca²⁺ cycling in cardiomyocytes, either through early-afterdepolarizations (EADs, mediated by re-activation of inactivated L-type Ca²⁺ channel) or delayed-afterdepolarizations (DADs, mediated by increased Na⁺/Ca²⁺ exchanger current), we hypothesized that Ca²⁺ trafficking alterations would rescue true drug-induced arrhythmias. To confirm the mechanistic properties of E4031-induced arrhythmia, a co-administration experiment with nifedipine, a Ca²⁺-channel inhibitor, was conducted. The observation that nifedipine is capable of rescuing E4031-induced arrhythmias, observed by both MEA and impedance, suggests that the erratic impedance trace reflects true drug-induced afterdepolarization-type arrhythmias. To confirm the ability of iPSC-CM to recapitulate arrhythmias *in vitro,* a broad panel of compounds clinically associated with TdP were examined, including cisapride, erythromycin, flecainide, ouabain, quinidine, sotalol and thioridazine. Irregular beating patterns were observed for each compound in a dose- and time-dependent manner. In comparison, the arrhythmic beats caused by ouabain lack the irregularity of beat and amplitude, instead inducing ventricular fibrillation-like arrhythmic "fasciculations." Terfenadine, which was withdrawn from the market due to TdP induction, blocks multiple cardiac ion channels (hERG, Na⁺ and Ca²⁺) yet *in vitro* detection of QT prolongation has been elusive. Although the hERG inhibition of terfenadine likely contributes substantially to its torsadogenic liability, the electrophysiological effects are masked by Ca²⁺ and Na⁺ channel inhibition and are thus not manifest readily in short term electrophysiology models. The arrhythmic effect was observed only after extended drug treatment (more than 12 hrs). Thioridazine, another multiple ion channel blocker, also requires longer term treatment to observe arrhythmia. Of note, thioridazine proarrhythmic responses have not previously been demonstrated using MEA devices because of the short duration of MEA experiments and highlights the importance of longer duration evaluation of arrhythmic risk. Additionally, an internal late-stage compound that caused TdP in non-human primates was examined by MEA and impedance. Like E4031, this compound caused a dose and time-dependent onset of arrhythmia in iPSC-CMs. The induced arrhythmia was also reversed by nifedipine treatment, again supporting these *in vitro* observations as true arrhythmias. As a negative control, compounds such as amoxicillin, aspirin, and captopril, which are devoid of cardiovascular side effects *in vivo,* did not show any alterations in beat patterns or arrhythmic beats, in human iPSC-CMs.

The ability to distinguish compounds that inhibit hERG in isolation, yet do not clinically induce TdP was examined using compounds such as verapamil and alfuzosin. These compounds exhibited *in vitro* results similar to the clinical experience, which includes QT prolongation, but not TdP. However, at concentrations substantially higher than employed *in vivo,* alfuzosin caused beat rate reduction and appearance of arrhythmic beats, similar to that of other hERG blockers. Thus, the rapid impedance measurements of human iPSC-CMs provides a substantial improvement in TdP prediction over basic hERG affinity and offer a novel paradigm for discovering human-relevant TdP *in vitro* at a very early stage in the pipeline.

The ability to detect arrhythmias using commercially available, quality controlled human iPSC-CMs with the high throughput system described herein could open new opportunities for studying human cardiovascular biology *in vitro.* However, if such a system is to achieve maximum utility within the drug discovery pipelines of the biotech and pharmaceutical industries, it must be capable of providing quantitative data that can be used to rank order new drug candidates by their predicted risk of causing cardiac arrhythmias. Therefore, as an initial effort to determine whether impedance measurements of drug-induced arrhythmias could provide a quantitative measure of proarrhythmic risk, we devised a method to calculate a "Predicted Proarrhythmic Score" (PPS). First, the lowest concentration of drug that resulted in greater than 20% irregular beats (IB₂₀) over one minute was determined. This threshold was selected empirically to optimize sensitivity and specificity. Next, the IB₂₀ was divided by the published value for a drug's maximal clinical efficacious plasma concentration Cₘₐₓ to arrive at the PPS. Thus, the PPS is an attempt to quantify the impedance-detected arrhythmia relative to a drug's efficacious exposure. PPS can be calculated by IB₂₀/Cₘₐₓ as in Table I. Using this calculation, PPS < 10 would indicate a level of concern for risk of induced arrhythmia.

Alternatively, the PPS may be calculated by dividing the Irregular Beat Ratio (IBR) by the Cₘₐₓ. Using this ratio, and based on clinical TdP incidence and our data, a PPS >100 was empirically chosen as threshold of concern for arrhythmogenesis. Cardioactive compounds like flecainide, ouabain, terfenadine, aconitine and quinidine show arrhythmia at concentrations less than the efficacious concentration and thus yield a high PPS. All other compounds with a high PPS were associated with TdP and/or induced arrhythmia at concentrations within 5-fold of efficacious exposure. Low risk compounds have a low PPS (<100). These include compounds devoid of QT prolongation and TdP liability (amoxicillin, aspirin, captopril, nifedipine, rofecoxib, verapamil), prolong QT duration but free of TdP (alfuzosin and ranolazine) or associated with very low risk of TdP (fluoxetine and amiodarone, Cardiovascular Research 58: 32-45, 2003.). The model system described herein demonstrates an improved accuracy, in particular the lack of false positives, when compared to either hERG screening and QT prolongation. Specifically ranolazine, verapamil, and moxifloxacin inhibit hERG, and alfuzosin, ranolazine, and moxifloxacin induce QT prolongation, yet are not torsadogenic in our model or in humans. Thus, the quantification of the proarrhythmia incidence and risk (based on effect versus efficacy) of known cardioactive compounds reveals a means to predict potential clinical effects. Combining the advanced technologies in micro-electrode bio-sensing and human iPSC-CMs together creates a unique opportunity to assess a drug candidate's effect on cardiac function in high-throughput and for the study of human cardiovascular biology. Fast sampling-rate impedance analysis reveals that iPSC-CMs recapitulate the cardiac contraction and relaxation of myocardium and confirms the expected effects of known cardioactive drugs. Arrhythmogenic drugs induced an impedance fingerprint of arrhythmia robustly and reproducibly at concentrations relevant to the clinic efficacy exposure. Drugs devoid of TdP and other severe arrhythmia, did not elicit any arrhythmia up to 10-fold of their maximal clinic exposure regardless of whether the compounds inhibit hERG or prolonged QT *in vivo,* demonstrating the increased accuracy of human iPSC-CMs to predict arrhythmogenic risk of drug candidates compared to current strategies employing hERG inhibition or prolonged QT as surrogates. Screening drug candidates for arrhythmic changes in human iPSC-CMs will facilitate a complete profiling of cardiac safety assessment and will improve the primary focus of cardiac safety evaluation: arrhythmogenicity detection.

### Definitions

The phrase "a" or "an" entity as used herein refers to one or more of that entity; for example, a compound refers to one or more compounds or at least one compound. As such, the terms "a" (or "an"), "one or more", and "at least one" can be used interchangeably herein.

As used in this specification, whether in a transitional phrase or in the body of the claim, the terms "comprise(s)" and "comprising" are to be interpreted as having an open-ended meaning. That is, the terms are to be interpreted synonymously with the phrases "having at least" or "including at least". When used in the context of a process, the term "comprising" means that the process includes at least the recited steps, but may include additional steps. When used in the context of a compound or composition, the term "comprising" means that the compound or composition includes at least the recited features or components, but may also include additional features or components.

As used herein, unless specifically indicated otherwise, the word "or" is used in the "inclusive" sense of "and/or" and not the "exclusive" sense of "either/or".

The term "optional" or "optionally" as used herein means that a subsequently described event or circumstance may, but need not, occur, and that the description includes instances where the event or circumstance occurs and instances in which it does not.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%.

The term "arrhythmia" as used herein generally refers to a condition in which there is abnormal electrical activity in the heart causing the heart to beat too fast or too slowly, in which the heartbeat may be regular or irregular. Some arrhythmias are life-threatening and can result in cardiac arrest and sudden death, whereas other arrhythmias are minor and can be regarded as normal variants. Arrhythmia can be classified by rate, e.g., normal, tachycardia (greater than 100 beats/minute), and bradycardia (less than 60 beats/minute), or mechanism, e.g., automaticity, reentry, and fibrillation. Ventricular arrhythmic beats are considered to be initiated by altered Ca²⁺ cycling in cardiomyocytes, either through early-afterdepolarizations (EADs, mediated by re-activation of inactivated L-type Ca²⁺ channel) or delayed-afterdepolarizations (DADs, mediated by increased Na⁺/Ca²⁺ exchanger current).

The term "proarrhythmia" as used herein refers to a new or more frequent occurrence of a pre-existing arrhythmia and is precipitated by anti-arrhythmic therapy. That is, it can be a side effect of anti-arrhythmic therapy with, for example, a cardiac glycoside.

The term "drug-induced arrhythmia" as used herein means arrhythmia caused, induced, or precipitated by or believed to be caused, induced or precipitated by a drug or medication.

The term "cardiomyocyte" or "cardiomyocytes" as used herein broadly refers to one or more muscle cells of the heart. The term cardiomyocyte includes smooth muscle cells of the heart, as well as cardiac muscle cells, which include also include striated muscle cells, as well as spontaneous beating muscle cells of the heart.

The term "field potential" means the measurement of potential difference between a pair of electrodes that arise from the change of "membrane potential" of a group of cardiomyocytes during the contraction and relaxation cycle. The "membrane potential" is sometimes used interchangeably with cell potential but is applicable to any lipid bilayer or membrane. Membrane potential (also called transmembrane potential or transmembrane potential difference or transmembrane potential gradient) is the electrical potential difference (measured by voltage) across a cell's plasma membrane. Membrane potential arises from the action of ion transporters embedded in the membrane which maintain viable ion concentrations inside the cell. The typical membrane potential of a cell arises from the separation of sodium ions from intracellular immobile anions across the membrane of the cell. This separation results from a concentration gradient of potassium ions by pumps or transporters. While there is an electric potential across the membrane due to charge separation, there is no actual measurable difference in the global concentration of positive and negative ions across the membrane. Thus, there is no measurable charge excess on either side. Cell membranes are typically permeable to only a subset of ionic species, including but not limited to potassium ions, chloride ions, bicarbonate ions, and others.

The term "pluripotent stem cell" as used herein means and includes the ability of a cell to differentiate into cell types of all three lineages or germ layers (viz. endoderm, ectoderm, and mesoderm). The term multipotent has a meaning understood in the art, and includes the ability of a cell to differentiate into multiple cell types. It is also understood that multipotent cells may be more restricted in their ability to differentiate than pluripotent cells. The term "iSCs", as used herein, refer to iPSCs or to induced multipotent stem cells (iMSCs). At times, the term "iPS" or "iPS cell" may be used instead of "iPSC"; similarly, at times the term "iMS" or "iMS cell" may be used instead of "iMSC". The methods and compositions described herein that are applicable to iPSCs are also applicable to induced stem cells (iSCs) and iMSCs.

The term "impedance" is used in a broad sense to indicate any collected, measured, and/or determined value that may include one or both of resistive and reactive components. Impedance data may include electrical parameter values that can be used to determine impedance (such as current and/or voltage values).

The term "MEA" as used herein means multi- or micro-electrode arrays and can be used to measure field potential and, when examining a homogenous and electrically coupled population of cells, mimic *in vivo* electrocardiograms. MEAs have been used previously to assess the general properties of stem cell derived cardiomyocyte action potential and the electrophysiological changes can be used as a surrogate measure of arrhythmias.

The term "TdP" as used herein refers to Torsades de Pointe.

The term "QT interval" as used herein refers to a measure of the time between the start of the Q wave and the end of the T wave in the heart's electrical cycle. The QT interval is thus dependent on the heart rate (the faster the heart rate, the shorter the QT interval). If abnormally prolonged or shortened, there is a risk of developing ventricular arrhythmias. QT interval prolongation may be measured utilizing assays that measure the disruption of ion channels. One example of such type of assay is a hERG channel assay. The hERG channel assay is described herein as associated with an indication of QT interval prolongation, and such assay is also a primary indicator of K⁽⁺⁾-channel blockage. hERG (which stands for "Human Ether-a-go-go Related Gene") encodes a potassium ion channel responsible for the repolarizing l[laquo]₍ᵣ₎ current in the cardiac action potential. This channel is sensitive to drug binding, which can result in decreased channel function and the so-called acquired long QT syndrome. Although there exist other potential targets for adverse cardiac effects, the vast majority of drugs associated with acquired QT prolongation are known to interact with the hERG potassium channel. One of the main reasons for this phenomenon is the larger inner vestibule of the hERG channel, thus providing more space for many different drug classes to bind and block this potassium channel.

The term "clinically efficacious concentration" or "Cₘₐₓ", as used herein means the plasma concentration to achieve the therapeutic efficacy in the clinic. Cₘₐₓ, maximum therapeutic plasma concentration.

The term "IBR" or "irregular beat ratio" as used herein means the incidence rate of irregular or arrhythmic beats and is calculated as the number of irregular beats/total beats in 1 min. IBR will be a value between 0-1 (or 0-100% if using % as the unit). The concentration of compound where the IBR ≥ 0.2 was determined (IB₂₀). IB₂₀ is equal to the lowest concentration that produces an IBR value ≥ 0.2 (≥ 20% irregular beats over one minute). This threshold was selected empirically to optimize sensitivity and specificity. The estimate for efficacy is the maximal clinical efficacious concentrations (Cₘₐₓ). In this application, Predicted Proarrhythmia Score (PPS) is calculated by two different ways: 1) the overall proarrhythmic risk was calculated by dividing the Cₘₐₓ by IB₂₀, which gives a ratio of the human *in vivo* exposure relative to the observed incidence of arrhythmia, to create a PPS, with the cut-off of >100 used as a level of concern (Table I) and 2) the overall proarrhythmic risk was calculated by dividing the IB₂₀ by the Cₘₐₓ, which gives a ratio of the observed incidence of arrhythmia relative to human *in vivo* exposure to create a PPS, with the cut-off of < 10 used as a level of concern (Table II). Using Cₘₐₓ/IB₂₀, based on clinical TdP incidence, a compound with a PPS>100 should be a sign of significant *in vivo* risk. For example, cardioactive compounds like ouabain and quinidine show arrhythmic beats at concentrations less than the efficacious concentration and thus have a high PPS. The 2^{nd} method of calculation (IB₂₀/Cₘₐₓ) is commonly used in the field of safety assessment and termed as the "Safety Margin", which indicates how close the concentration that causes the safety concern to the concentration of therapeutic efficacy in the clinic. The larger the safety margin is, the lower the risk for drug toxicity. Conversely, using the 1^{st} method, (Cₘₐₓ/IB₂₀), the larger the PPS value is, the higher the risk is for drug toxicity.

The term "high throughput" as used herein means an assay design that allows easy screening of multiple samples simultaneously and capacity for robotic manipulation. Another desired feature of high throughput assays is an assay design that is optimized to reduce reagent usage, or minimize the number of manipulations in order to achieve the analysis desired. Examples of assay formats include 96-well or 384-well plates. It is well known in the art that as miniaturization of plastic molds and liquid handling devices are advanced, or as improved assay devices are designed, greater numbers of samples may be performed using the design of the present invention. In one embodiment, the cells are cultured and analyzed in the micro-titer plates containing a plurality of wells such as 96- or 386-well plates.

The term "drug discovery setting" as used herein means any setting wherein the purpose of employing the methods described herein is to identify pharmaceutically acceptable drugs. For example, any setting wherein the methods described herein are used to identify possible drug molecules that have low risk of inducing cardiotoxicity as measured by incidence of drug induced arrhythmia.

Technical and scientific terms used herein have the meaning commonly understood by one of skill in the art to which the present invention pertains, unless otherwise defined. Reference is made herein to various methodologies and materials known to those of skill in the art. Standard reference works setting forth the general principles of pharmacology include Goodman and Gilman's The Pharmacological Basis of Therapeutics, 10th Ed., McGraw Hill Companies Inc., New York (2001). Any suitable materials and/or methods known to those of skill can be utilized in carrying out the present invention. However, preferred materials and methods are described. Materials, reagents and the like to which reference are made in the following description and examples are obtainable from commercial sources, unless otherwise noted.

### EXAMPLES

### METHODS

### Chemicals

*In vitro* reagents were purchased from Sigma Chemical Co. (St Louis MO).

### Methods

**Cell Culture.** hiPSC-derived cardiomyocytes (iCells) from Cellular Dynamics International (CDI) were thawed in Plating Media (CDI and plated as single cells onto 0.1% Gelatin (Sigma)-coated, 6-well tissue-culture plates (Corning) at a density of 2.2 - 2.7 x 10⁶ cells per well. Cells were cultured for 3-5 d at 37 °C, 7% CO₂ prior to re-plating onto xCELLigence® 96-well cardio e-plates (ACEA/Roche Applied Sciences) or microelectrode arrays (MEAs, Multichannel Systems). The media was changed every 2 d after plating using Maintenance Media (CDI), pre-warmed to 37 °C to minimize the temperature shock on cells.

### Overview

The new RTCA cardio system allows the monitoring of cell experiments over a longer timeframe and measures in parallel the contraction (beating) of the cells. At each measurement time point the Cell Index and underlying Frequency and Amplitude of the contraction is measured.

### System

The xCELLigence® RTCA Cardio station are placed in incubator set at 7% CO2. Incubator is available at the customer site. The following components are tested:

| | **Type** | **Specification** |
|---|---|---|
| RTCA Cardio Analyzer | Hardware for extracellular Reading and signal processing | Prototype system |
| RTCA Cardio Station | Station for Impedance reading of the E-Plates | Prototype system |
| RTCA Cardio Software V0.X | | Prototype system |
| RTCA Control Unit | | Prototype system |
| E-Plate Cardio | | Prototype devices |

**xCELLigence® cardio e-plates and MEA Re-plating.** iCells cultured in 6-well plates were harvested by twice washing in dPBS (GIBCO) then lifted with 0.5% Trypsin-EDTA (GIBCO), incubated at 37 °C, 7% CO₂. Trypsin was quenched with Maintenance Media and cells were collectively centrifuged at 69g for 5 minutes and resuspended in Maintenance Media to yield the target density. For cardio e-plate reseeding, cells were plated at a target density of 5 x 10⁴ per well, estimating 80% seeding efficiency. Cardio e-plates were coated with 0.1% Gelatin for 3 h at 37 °C. Background impedance measurements for cardio e-plates were made with the final volume of media equilibrated to 37 °C but without cells, immediately prior to reseeding the cells. The cells were monitored every 1 h by a 20 s sweep duration by the xCELLigence® RTCA cardio system at its maximum sampling rate (77 Hz) prior to initiating experimentation.

MEAs were prepared according to manufacturer guidelines. Briefly, microelectrodes in 6-well MEA dishes were coated with 2 µL Fibronectin (Sigma) diluted 1:20 and incubated at 37 °C for 3 h. Cells were reseeded at the target density of 3 x 10⁴ cells in a 2 µL delivery to microelectrodes and incubated at 37 °C, 7% CO2 for 3 h prior to filling each well with Maintenance Media. The media of cardio e-plates and MEAs were changed every 2 d thereafter, using Maintenance Media warmed to 37 °C. Cells were cultured for 3-7 d prior to conducting an experiment on cardio e-plates or MEAs.

**xCELLigence® and MEA Experimentation.** Compound stocks were prepared in DMSO or dH₂O at 1000 fold the highest tested concentration. For xCELLigence® experimentation, compound stocks were serially diluted in Maintenance Media in a separate 96-well tissue culture plate (Corning) at twice the target concentration. The dilution plate was incubated and equilibrated to 37 °C prior to cell exposure. Half the present media volume was removed from the cardio e-plate and replaced with an equal volume of the respective 2x greater compound concentration, yielding the final target concentration in the respective well. All compounds were tested as n ≥ 3 on multiple cardio e-plates. The xCELLigence® monitored changes to the beating for periodic 20 s sweep durations at 77 Hz.

For MEA experimentation, compound stocks were serially diluted in Maintenance Media in eppendorf tubes at 20x the target concentrations. The wells of the MEAs had half the volume in each well replaced with fresh warm Maintenance Media, immediately prior to experimentation so as to be consistent with the media replacement for xCELLigence® experimentation. MEAs were allowed a 15 min equilibration period within the MEA recorder system (Multichannel Systems), prior to compound exposure. The temperature was maintained at 37 °C and a constant air flow of 95% O₂, 5% CO₂, was perfused over the MEAs. Compound additions were made in serially increasing additions, recording for 15 min at each concentration. All compounds were tested as n ≥ 4 wells. Each 6-well MEA possessed at least one well as the time-matched vehicle (DMSO or dH₂O) control.

**Data Analysis.** Data were first analyzed by the built-in analysis modules of xCELLigence® and MEAs systems, initial results were then exported into in-house developed Microsoft Excel templates for further semi-auto analysis. Arrhythmic contractions/beats were defined in both xCELLigence® and MEA recordings as those with largely reduced amplitude and occurred pre-maturely prior to an anticipated regular contraction/beat. To calculate the "Predicted Proarrhythmic Score" (PPS), we first determined a "Irregular Beat Ratio" (IBR). The IBR was defined as the ratio of arrhythmic beat counts to the total number of beats over one minute. IBR values for each concentration at each selected time-point was averaged from 3 to 5 e-plates. The lowest concentration of drug, that generated a IBR value ≥ 0.2 was determined and termed "IB₂₀." Finally, the maximal therapeutic plasma concentration (Cₘₐₓ) was divided by the IB₂₀ to arrive at the PPS. A PPS >100 is a level of concern for drug-induced arrhythmia.

To assess the treatment-related change in a parameter other than IBR, the measurement after treatment was normalized to the baseline (pre-drug) level, and compared to that obtained from the time-matched vehicle control group. Data were expressed as the mean ± S.E., and the statistical significance of the differences was analyzed using a two sample student's *t*-test (Excel 2003 SP3), assuming equal variances, with a p-value < 0.05.

### Protocol and Performance Criteria

Protocol:
1. iCells are allowed to attach on 6-well plates in plating media
2. The first media change occurs 48 hours after attachment and cells are replenished with iCell maintenance media.
3. Cells are dissociated and plated on fibronectin coated e-plates at 50K cells/well. The cells are monitored for up to 3 days with a measurement rate of 1 measurement/hour. Thereafter the cells are washed and again monitored for 2-3 days with a measurement rate of 1 measurement/hour.
4. Compounds are applied to wells in triplicates at 24 hours or longer after seeding, until the stable beating is reached in all wells.
5. The impedance measurement is initialized upon the completion of cell-seeding with a 30-second measurement interval throughout the whole experiment. The measurement rate after the compound addition is 60 measurements/hour for the first hour after treatment and 12 measurements/hour for the second and third hour after treatment and 2 measurements/hour for the following 24 hours. For the monitoring 24 to 72 hours after the treatment, the measurement rate is 1/h
6. After compound application, the contraction and viability are monitored up to 72 hours. In selected examples, compounds may be studied for a longer period of time.

Cardio-plate Layout:

| | 1 | 2 | 3 | 4 | 5 | 6 | 7 | 8 | 9 | 10 | 11 | 12 | Comment |
|---|---|---|---|---|---|---|---|---|---|---|---|---|---|
| A | 1.1 | 2.1 | 3.1 | 1.1 | 2.1 | 3.1 | 1.1 | 2.1 | 3.1 | D | D | D | |
| B | 1.2 | 2.2 | 3.2 | 1.2 | 2.2 | 3.2 | 1.2 | 2.2 | 3.2 | S | S | S | |
| C | 1.3 | 2.3 | 3.3 | 1.3 | 2.3 | 3.3 | 1.3 | 2.3 | 3.3 | n | n | n | |
| D | 1.4 | 2.4 | 3.4 | 1.4 | 2.4 | 3.4 | 1.4 | 2.4 | 3.4 | 2 | 3 | 3 | |
| E | 1.5 | 2.5 | 3.5 | 1.5 | 2.5 | 3.5 | 1.5 | 2.5 | 3.5 | 2 | 3 | 3 | |
| F | 1.6 | 2.6 | 3.6 | 1.6 | 2.6 | 3.6 | 1.6 | 2.6 | 3.6 | 2 | 3 | 3 | |
| G | 1.7 | 2.7 | 3.7 | 1.7 | 2.7 | 3.7 | 1.7 | 2.7 | 3.7 | 2 | 3 | 3 | |
| H | 1.8 | 2.8 | 3.8 | 1.8 | 2.8 | 3.8 | 1.8 | 2.8 | 3.8 | 2 | 3 | 3 | |

iCell cardiomyocytes on gelatin
1.1: compound 1 in concentration 1
2.1: compound 2 in concentration 2
x.y: compound x in concentration y
D: DMSO
S: solvent
N: negative control

### Reference Methods/Instruments:

The compounds used for RTCA Cardio experiments are screened with the Microelectrode Array (MEA) and for cytotoxicity using ATP as the benchmark.

The foregoing invention has been described in some detail by way of illustration and example, for purposes of clarity and understanding. It will be obvious to one of skill in the art that changes and modifications may be practiced within the scope of the appended claims. Therefore, it is to be understood that the above description is intended to be illustrative and not restrictive. The scope of the invention should, therefore, be determined not with reference to the above description, but should instead be determined with reference to the following appended claims, along with the full scope of equivalents to which such claims are entitled.

## Claims

1. An in vitro high throughput method of determining risk of drug-induced arrhythmia comprising:
a) providing cardiomyocytes in a 96-well tissue culture plate with an interdigitated electrode sensor array capable of rapid sampling of impedance;
b) contacting said cardiomyocytes with said drug;
c) detecting beat rate irregularity by monitoring either cell contraction or field potential;
d) calculating the IBR (irregular beat ratio) wherein a IBR of less than or equal to 0.2 is used to designate a low risk of arrhythmia;
wherein the cardiomyocytes are produced from an induced pluripotent stem cell source.

2. The method of claim 1, wherein the cardiomyocytes are of dog, monkey, rat, rabbit, or human origin.

3. The method of any one of claims 1-2, wherein the incidence of arrhythmia is detected by monitoring changes in cell field potential.

4. The method of any one of claims 1-3, wherein the cell contraction is monitored by measuring impedance.

5. The method of claim 4, wherein the cell contraction is monitored by measuring impedance with a sampling rate of about every 12.9 milliseconds.

6. The method of any one of claims 1-5, wherein the detection of arrhythmia comprises monitoring increases, decreases, or irregularity of beat rate rhythm.

7. The method of claim 6, wherein the irregularity of beat rate rhythm is indicative of Torsades de Pointes, prolongation of QT, or disruption of a cardiac ion channel.

8. The method of any one of claims 1-7, further comprising:
e) calculating the ratio of the IB₂₀ relative to Cₘₐₓ, resulting in a PPS (predicted proarrhythmic score) or calculating the ratio of the IB₂₀ relative to Cₘₐₓ, resulting in a PPS.

9. The method of claim 8, wherein a PPS of less than or equal to 100 is used to designate a low risk of arrhythmia.

10. The method of any one of claims 8-9, further comprising:
f) comparing the result of step e) to that of a known arrhythmia inducing drug.

11. The method of any one of claims 1-10, wherein the method of determining risk of drug-induced arrhythmia is conducted to screen molecules in a drug development setting.

## Patentansprüche

1. In-vitro-Hochdurchsatzverfahren zum Bestimmen des arzneimittelinduzierten Arrhythmierisikos, umfassend:
a) Bereitstellen von Kardiomyozyten in einer 96-Well-Gewebekulturplatte mit einer Sensoranordnung mit ineinandergreifenden Elektroden, die zur schnellen Impedanzabtastung in der Lage ist;
b) Inkontaktbringen der Kardiomyozyten mit dem Arzneimittel;
c) Feststellen der Unregelmäßigkeit der Herzschlagfrequenz durch Überwachen von entweder der Zellkontraktion oder des Feldpotenzials;
d) Berechnen des Verhältnisses unregelmäßiger Herzschläge (irregular beat ratio, IBR), wobei ein IBR von weniger als oder gleich 0,2 verwendet wird, um ein niedriges Arrhythmierisiko zu bezeichnen;
wobei die Kardiomyozyten aus einer Quelle für induzierte pluripotente Stammzellen erzeugt werden.

2. Verfahren nach Anspruch 1, wobei die Kardiomyozyten von Hunden, Affen, Ratten, Kaninchen oder Menschen stammen.

3. Verfahren nach einem der Ansprüche 1 bis 2, wobei das Auftreten der Arrhythmie durch Überwachen von Veränderungen des Feldpotenzials der Zelle festgestellt wird.

4. Verfahren nach einem der Ansprüche 1 bis 3, wobei die Zellkontraktion durch Impedanzmessung überwacht wird.

5. Verfahren nach Anspruch 4, wobei die Zellkontraktion durch Impedanzmessung mit einer Abtastrate von etwa alle 12,9 Millisekunden überwacht wird.

6. Verfahren nach einem der Ansprüche 1 bis 5, wobei die Feststellung der Arrhythmie das Überwachen von Zunahmen, Abnahmen oder Unregelmäßigkeit des Herzschlagrhythmus umfasst.

7. Verfahren nach Anspruch 6, wobei die Unregelmäßigkeit des Herzschlagrhythmus auf Torsades de Pointes, verlängertes QT-Intervall oder Unterbrechung eines kardialen Ionenkanals hindeutet.

8. Verfahren nach einem der Ansprüche 1 bis 7, ferner umfassend:
e) Berechnen des Verhältnisses des IB₂₀ relativ zu Cₘₐₓ, was einen prognostizierten proarrhythmischen Score (PPS) ergibt, oder Berechnen des Verhältnisses des IB₂₀ relativ zu Cₘₐₓ, was einen PPS ergibt.

9. Verfahren nach Anspruch 8, wobei ein PPS von weniger als oder gleich 100 verwendet wird, um ein niedriges Arrhythmierisiko zu bezeichnen.

10. Verfahren nach einem der Ansprüche 8 bis 9, ferner umfassend:
f) Vergleichen des Resultats von Schritt e) mit dem eines bekannten arrhythmieinduzierenden Arzneimittels.

11. Verfahren nach einem der Ansprüche 1 bis 10, wobei das Verfahren zur Feststellung des arzneimittelinduzierten Arrhythmierisikos durchgeführt wird, um Moleküle im Rahmen einer Arzneimittelentwicklung zu testen.

## Revendications

1. Procédé *in vitro* à haut débit de détermination de risque d'arythmie induite par un médicament consistant à :
a) utiliser des cardiomyocytes dans une plaque de culture de tissus à 96 puits avec une barrette de capteurs à électrodes interdigitées permettant d'échantillonner rapidement l'impédance ;
b) mettre en contact lesdits cardiomyocytes avec ledit médicament ;
c) détecter une irrégularité dans la fréquence du rythme cardiaque en surveillant soit la contraction des cellules, soit le potentiel de champ ;
d) calculer la fréquence du rythme cardiaque irrégulier (IBR), une IBR inférieure ou égale à 0,2 étant utilisée pour désigner un risque faible d'arythmie ;
dans lequel les cardiomyocytes sont produits à partir d'une source de cellules souches pluripotentes induites.

2. Procédé selon la revendication 1, dans lequel les cardiomyocytes ont pour origine un chien, un singe, un lapin ou un humain.

3. Procédé selon l'une quelconque des revendications 1 et 2, dans lequel l'incidence d'une arythmie est détectée par la surveillance des variations dans le potentiel de champ des cellules.

4. Procédé selon l'une quelconque des revendications 1 à 3, dans lequel la contraction des cellules est surveillée par la mesure de l'impédance.

5. Procédé selon la revendication 4, dans lequel la contraction des cellules est surveillée par la mesure de l'impédance avec une fréquence d'échantillonnage d'environ toutes les 12,9 millisecondes.

6. Procédé selon l'une quelconque des revendications 1 à 5, dans lequel la détection d'une arythmie comprend la surveillance des augmentations, des diminutions ou de l'irrégularité du rythme de la fréquence cardiaque.

7. Procédé selon la revendication 6, dans lequel l'irrégularité du rythme de la fréquence cardiaque indique des Torsades de Pointes, un allongement de l'intervalle QT, ou une rupture d'un canal ionique cardiaque.

8. Procédé selon l'une quelconque des revendications 1 à 7, consistant en outre à :
e) calculer le rapport IB₂₀/Cₘₐₓ, permettant d'obtenir un PPS (score proarythmique prédit) ou calculer le rapport IB₂₀/Cₘₐₓ, permettant d'obtenir un PPS.

9. Procédé selon la revendication 8, dans lequel un PPS inférieur ou égal à 100 est utilisé pour désigner un risque faible d'arythmie.

10. Procédé selon l'une quelconque des revendications 8 à 9, consistant en outre à :
f) comparer le résultat de l'étape e) avec celui d'un médicament connu pour induire une arythmie.

11. Procédé selon l'une quelconque des revendications 1 à 10, ledit procédé de détection de risque d'arythmie induite par un médicament étant mis en oeuvre pour cribler des molécules dans le cadre du développement de médicaments.
